# EUROPEAN PATENT APPLICATION

(11) **EP 4 678 486 A1**
(43) Date of publication of application: **14.01.2026**
(21) Application number: 24187122.7
(22) Date of filing: 08.07.2024
(51) Int. Cl.: B60R 25/25, G07C 9/00, A61B 5/08, B60K 28/06

(54) **VEHICLE ACCESS CONTROL SYSTEM**

(71) Applicant: trinamiX GmbH, 67063 Ludwigshafen am Rhein (DE)
(72) Inventor: Lehnert, Tibor Peter, 67059 Ludwigshafen am Rhein (DE); Oeguen, Celal Mohan, 67059 Ludwigshafen am Rhein (DE); Loeffler, Jan Gerrit, 60431 Frankfurt (DE); Seidel, Sebastian, 67059 Ludwigshafen am Rhein (DE); Sheeran, Bridget, 67059 Ludwigshafen am Rhein (DE); Unruh, Tobias, 35781 Weilburg (DE); Baumgartner, Tobias, 67059 Ludwigshafen am Rhein (DE); Schmidt, Felix, 67059 Ludwigshafen am Rhein (DE)
(74) Representative: BASF IP Association

(57) **Abstract**

The invention is in the field of access control systems for vehicles. It relates to a portable access control device for a vehicle comprising:
a) a spectroscopy module for acquiring spectroscopic data of a person,
b) a processor configured to generate an access signal using the spectroscopic data, and
c) a communication interface to transmit the access signal to the vehicle.

## Description

The invention is in the field of access control systems for vehicles. The invention relates to a portable access control device for a vehicle, an access control system for integration into a vehicle, method for controlling access to a vehicle, and a non-transient computer-readable medium including instructions that, when executed by one or more processors, cause the one or more processors to perform a method for controlling access to a vehicle.

### Background

Traffic security has been tremendously increased by technical safety systems built into vehicles. However, the human driver remains a serious security threat, in particular under the influence of intoxicants like alcohol. Keeping humans under the influence from driving a vehicle can thus increase traffic security.

EP 1703045 A1 suggests a car key with a breath component detection means for detecting alcohol. Such component requires the driver to breath into the key which is hygienically questionable and takes a while until the measurement is finished. Hence, such a system suffers from low user acceptance.

It was hence the object of the present invention to provide a reliable and comfortable vehicle access control system to detect driver under the influence.

### Summary

In one aspect the invention relates to portable access control device for a vehicle comprising:
a) a spectroscopy module for acquiring spectroscopic data of a person,
b) a processor configured to generate an access signal using the spectroscopic data, and
c) a communication interface to transmit the access signal to the vehicle.

In another aspect the invention relates to an access control system for integration into a vehicle comprising:
a) a communication interface to receive an access signal from a portable access control device, wherein the access signal comprises data derived from a spectroscopic measurement,
b) a processor configured to generate an unlock signal using the access signal, wherein the unlock signal generation involves a determination of the eligibility to drive of the person seeking access to the vehicle from the access signal, and
c) a communication interface for transferring the unlock signal to a locking mechanism of the vehicle.

In another aspect the invention relates to a method for controlling access to a vehicle comprising:
a) receiving spectroscopic data of a person seeking access to the vehicle from a spectroscopy module integrated into a portable access control device,
b) determining a concentration of a body substance from the spectroscopic data,
c) generating an unlock signal using the concentration of the body substance, and
d) transferring the unlock signal to a locking mechanism of the vehicle.

In another aspect the invention relates to a non-transient computer-readable medium including instructions that, when executed by one or more processors, cause the one or more processors to perform a method comprising:
a) receiving spectroscopic data of a person seeking access to the vehicle from a spectroscopy module integrated into a portable access control device,
b) determining a concentration of a body substance from the spectroscopic data,
c) generating an unlock signal using the concentration of the body substance, and
d) transferring the unlock signal to a locking mechanism of the vehicle.

The advantage of the present invention is that the concentration of a body substance of a driver seeking access to a vehicle can be determined more accurately within short measurement time. In this way, the safety is increased as a compromised fitness to drive can be quickly and easily detected, for example due to intoxicants such as alcohol or drugs, due to a health problem, for example low sugar concentration of a diabetes patient, or due to fitness problems like dehydration. At the same time the measurement is more hygienical, it can even be done without any contact. In any case, the vehicle access control system is very easy to clean. This enhances the user experience and thereby the user acceptance. A higher user acceptance can be expected leading to an increased safety level. The required hardware is simple and can be produced at high volumes at low prices. Low-weight hardware can be used which consumes little energy. Spectroscopy further allows determination of multiple body substances or the merger with other data which allows an even further security increase.

The term "access control device" may refer to any type of device that can be used to control access to a vehicle or its compartments. These devices may be designed to only allow authorized individuals to enter the vehicle or start the vehicle while refusing unauthorized individuals. The term "portable access control device" may refer to an access control device which is not permanently attached to a vehicle, and which may have a format and a weight which allows an average person to carry the portable access control device. Examples for portable access control device include a physical key, i.e. a key which can be inserted into a lock cylinder to unlock or start the vehicle; a key fob, i.e. devices to communicate with the vehicle, for example using radio frequency identification (RFID) technology, and unlock or start the vehicle; a mobile computing device, for example a smartphone, a smartwatch or a tablet computer, which communicate with the vehicle, for example via RFID or Wi-Fi, to unlock or start the vehicle.

The portable access control device comprises a spectroscopy module. The term "spectroscopy module" may refer to an apparatus which is capable of recording spectroscopic data of a person. A spectroscopy module may comprise:
- an optical element configured for separating incident optical radiation provided by the measurement driver into a spectrum of constituent wavelength components;
- a photosensor comprising at least one photosensitive region configured for receiving the optical radiation from the optical element, wherein the photosensor is configured for generating at least one photosensor signal dependent on an illumination of the photosensitive region by the optical radiation;
- a processor to process the photosensor signals into a spectrum.

The term "optical element" may refer to an arbitrary element configured for influencing optical radiation. The optical element may be configured for at least one of at least partially dispersing the optical radiation, at least partially filtering the optical radiation, at least partially reflecting the optical radiation, e.g. diffusely or directly, at least partially deflecting the optical radiation, at least partially transmitting the optical radiation and at least partially absorbing the optical radiation. The optical element may comprise at least one of a prism, a grating, a beam splitter, or an interferometer, for example a Michelson interferometer. The optical element may be configured for being used in mobile applications, for example for being used in handheld spectrometer devices and/or in spectrometer devices comprised by electronic communication devices, such as a smartphone or a tablet. As another example, the optical element may comprise at least one optical filter element. The optical filter element may be configured for filtering the optical radiation or more specifically at least one selected spectral range of the optical radiation. The optical filter element may specifically be positioned in a light path before the photosensor. As an example, the portable spectrometer may comprise a plurality of photosensors, for example 5 to 20, such as 8 to 12. The photosensors may be arranged as pixels in an array or in a matrix. The portable spectrometer may comprise a plurality of optical filter elements. An optical filter element may be positioned in a beam path before a photosensor. The optical filter elements may be transmissive at different wavelengths of different wavelength regions. For example, each photosensor may be positioned behind an optical filter with regard to the beam path, wherein each optical filter is transmissive at different wavelength or different wavelength region to the other optical filters. Such a setup allows a making the spectroscopy module very compact so it can easily be integrated into an access control device.

The spectroscopy module may comprise one or more than one photosensor. The photosensor may comprise at least one photosensitive region. The photosensitive region may be configured for receiving the optical radiation from the optical element. The photosensor may be configured for generating at least one photosensor signal dependent on an illumination of the photosensitive region by the optical radiation. The term "sensor" may refer to a device configured for detecting at least one condition or for measuring at least one measurement variable. The sensor may be capable of generating at least one signal, such as a measurement signal, which is a qualitative or quantitative indication of the measurement variable and/or measurement property, e.g. of an illumination of the sensor or a part of the sensor. The signal may be or comprise an electrical signal, such as a current, specifically a photocurrent. The term "photosensor" may refer to a sensor or a detector configured for detecting or measuring optical radiation, such as for detecting an illumination and/or a light spot generated by at least one light beam, e.g. by using the photoelectric effect. The photodetector may comprise at least one substrate. As an example, a single photosensor may be a substrate with at least one single photosensitive region, which generates a physical response, e.g. an electronic response, to the illumination for a given wavelength range.

The term "photosensitive region" may refer to a unit of the photosensor, specifically to a spatial area or volume being part of the photosensor, configured for being illuminated, or in other words for receiving optical radiation, and for generating at least one signal, such as an electronic signal, in response to the illumination. The photosensitive region may be located on a surface of the photosensor. The photosensitive region may specifically be a single, closed, uniform photosensitive region. However, other options may also be feasible.

The spectroscopy module may comprise at least one light emitting element configured for emitting illumination light for illuminating the driver in order to generate detection light from the driver. The light emitting element may be an incandescent lamp, for example a tungsten filament lamp or a tungsten halogen lamp, a light-emitting diode (LED), a laser diode, a gas-discharge lamp, for example a xenon lamp, a mercury vapor lamp, or a deuterium lamp.

The term "light" may refer to electromagnetic radiation in one or more of the infrared, the visible and the ultraviolet spectral range. The term "ultraviolet spectral range" may refer to electromagnetic radiation having a wavelength of 1 nm to 380 nm, preferably of 100 nm to 380 nm, for example 280 nm to 315 nm (UV-B) or 315 nm to 380 nm (UV-A). Further, in partial accordance with standard ISO-21348 in a valid version at the date of this document, the term "visible spectral range" may refer to a spectral range of 380 nm to 760 nm. The term "infrared spectral range" (IR) may refer to electromagnetic radiation of 760 nm to 1000 µm, wherein the range of 760 nm to 1.5 µm is usually denominated as "near infrared spectral range" (NIR) while the range from 1.5 µ to 15 µm is denoted as "mid infrared spectral range" (MidIR) and the range from 15 µm to 1000 µm as "far infrared spectral range" (FIR). Preferably, light used for the typical purposes of the present invention is light in the infrared (IR) spectral range, more preferred, in the near infrared (NIR) and/or the mid infrared spectral range (MidIR), especially the light having a wavelength of 750 nm to 3 µm, for example 780 nm to 1.4 µm or 1.4 µm to 2.5 µm. These wavelength regions are particularly suitable for obtaining material properties of a driver.

The spectroscopy module may comprise a processor to process the photosensor signals into spectroscopic data, for example an infrared spectrum. The processor may output the spectroscopic data, for example to an interface for further processing or to a user interface. The processor may further be configured to apply a chemometric model and output the concentration of a body substance obtained by the chemometric model. The spectroscopy module may further comprise a memory. The memory may be configured to store the chemometric model. The memory may be configured to store spectroscopic data.

The spectroscopy module may be positioned such that it can illuminate the person with light. The spectroscopy module may be positioned such that it can receive light from the person. The spectroscopy module may be attached to the outside of the access control device, or it may be inside the access control device. In particular in the latter case, the access control device may comprise a hole or a window through which the light can propagate. A window may be out of glass, for example quartz glass or an alkali-aluminosilicate sheet glass like Gorilla glass, a fluoride salt, for example calcium fluoride, or an aluminum oxide crystal, for example a sapphire.

The term "spectroscopic data" may refer to data associated with a spectroscopic measurement of a driver, in particular with optical spectroscopic measurement of the driver. The spectroscopic data may be received from the spectroscopy module of the present invention. The spectroscopic data may be received directly from a spectroscopy module or indirectly, i.e. from a storage device to which the spectroscopic data have been stored after the measurement. A spectroscopic measurement may be triggered by a predefined event, for example when the vehicle is switched on, before the engine is started, or after a certain time period. A spectroscopic measurement may be triggered when a measurement trigger event occurs. A measurement trigger event may be a situation in which an indicator indicates the necessity for a spectroscopic measurement necessary. A measurement trigger event may occur when an indicator indicate that the driver's fitness to drive is potentially compromised, for example due to intoxicants such as alcohol or drugs, due to a health problem, for example low sugar concentration of a diabetes patient, or due to fitness problems like dehydration. The measurement trigger event may be determined using person data and/or environmental data. For example, the person data and/or environmental data may indicate an increased likelihood that the driver's fitness to drive the vehicle are compromised, such as slow pupil reflex recorded by an optical camera, unusual movement patterns recorded by a pressure sensor, or certain voice characteristics recorded by a microphone. Triggering a spectroscopic measurement in such cases may be particularly useful if the body substance is used for access control of the vehicle, for example to keep drunk drivers from driving without burdening obviously sober drivers with a measurement.

The spectroscopic measurement may be made at various body parts of the person, for example the face, in particular the lips, the arm, the hand. Parts of the face are advantageous such as lips, ear lobe or forehead. In particular, lips have a thin epidermis, no hair follicles, few sweat glands, may blood vessels, and no melanin. The access control device may comprise a mouthpiece to facilitate lip measurement. The mouthpiece may be permanently attached to the access control device or be a disposable part which can be replaced, for example after each measurement. The access control device may be configured to enable measurement of both lips at the same time, for example by a mouthpiece splitting the light beam and directing it to both the upper and the low lip. The spectroscopic measurement may be made at parts of the hand, for example the palm, the back of the hand, one or multiple fingers, such as the thumb, the forefinger, the long finger, the ring finger or auricular finger. The spectroscopic measurement may be made in direct contact with the person or in close proximity, for example with a distance of less than 10 cm or less than 5 cm between driver and spectrometer device.

Spectroscopic data may be or may comprise one or more than one spectrum. The term "spectrum" may refer to a data structure in which several intensity values or values derived thereof such as absorbance of radiation are associated with wavelengths or wavelength ranges of the radiation. The wavelength or wavelength ranges may be those described above. The data structure may be a vector, wherein each element represents an intensity and the position in the vector represents a certain wavelength or wavelength range, so the value at a certain position represents the intensity of that wavelength or wavelength range. The data structure may be a vector or matrix containing value pairs, wherein one value represents the wavelength or wavelength range and the other value the intensity at this wavelength or wavelength range. The spectrum recorded by the spectrometer may be corrected by calibration coefficients to compensate for sensor imperfections or drifts. The spectrum may represent the absorbance or transmittance of radiation after having penetrated the skin of the person.

The spectroscopic data may be used to determine a concentration of a body substance such as the blood alcohol concentration. The determination may be accomplished by a chemometric model which receives spectroscopic data as input and outputs the concentration of a body substance. The concentration of a body substance may be used to generate an unlock signal, i.e. a signal directed to the locking hardware causing the locking hardware to unlock. For example, the unlock signal may be generated only if the concentration of a body substance is above or below a threshold. The concentration of the body substance may be the blood alcohol concentration, wherein the unlock signal is only generated if the blood alcohol concentration is below the allowed threshold, for example below 0.05 %. Another example may be the hydration level which needs to be above a threshold to mitigate the risk of sudden feeling of faintness. The determination of the concentration of a body substance may be performed on the access control device or on the access control system.

The access control device generates an access signal using the spectroscopic data. The access signal may comprise the spectroscopic data or parts thereof. In this case, the access control system may determine the concentration of a body substance from the spectroscopic data. The access signal may comprise the concentration of a body substance. In this case, the access control device may determine the concentration of a body substance from the spectroscopic data. The access signal may comprise a flag indicating the access rights of a person, for example the eligibility to access the vehicle or the functionalities of the vehicle which the person is eligible to use, for example start the engine or use the entertainment system. In this case, the access control device may determine the concentration of a body substance from the spectroscopic data and further determine if the concentration of a body substance qualifies the person to access the vehicle. This may be particularly suitable for the case that the access control device is a portable computing device such as a smartphone.

The access signal may be a Boolean value indicating whether the access can be granted or not. The access signal may be a numeric value, for example classifier indicating the extent of access which can be granted to the person. The access signal may be generated by determining if the concentration of a body substance is above or below a preset threshold. The determination of the access signal may involve region-specific settings, for example a country or state-specific concentration of a body substance threshold. The region-specific settings may be obtained from a storage medium taking into account the geographic location of the vehicle, for example obtained from a GPS system. The determination of the access signal may involve person data, for example the person's age to determine an age-specific threshold of blood alcohol concentration. The determination of the access signal may involve personalized person data, for example a personalized threshold of blood alcohol concentration which may be lower than the general threshold, for example due to a court order as a consequence of a prior driving under the influence. Personalized person data may be selected from a database using the person identity obtained from person identification as described above.

The access signal may be used for geofence lockout, for example prevent the vehicle from leaving a designated area, for example a home or highways, if a preset concentration of a body substance is exceeded; for passive alert, for example discreetly notify emergency contacts or roadside assistance if a preset concentration of a body substance is exceeded; for adapting autonomous driving functionality, for example, increase distance kept to vehicles driving in front and increase break system pressure to allow for more effective breaking and avoid accidents due to reduced reaction time if the concentration of a body substance is within a preset range; for data logging, for example maintain a discreet log of concentration of a body substance readings for personal health tracking or potential use by law enforcement; for determining eligibility, for example restrict driving privileges based on concentration of a body substance for individuals with prior driving under the influence convictions or for novice persons; for insurance premium adjustments, for example to adjust insurance premiums based on concentration of a body substance measurement history to encourage responsible driving behavior; for emergency response decisions, for example to improve decision making of law enforcement and medical personnel, taking into account concentration of a body substance levels of individuals involved in accidents or medical emergencies; for real-time fleet monitoring, for example an alert fleet managers to elevated concentration of a body substance readings, allowing for immediate intervention such as contacting the person, dispatching a replacement, in particular for commercial vehicles; for route restriction, for example automatically reroute vehicles driven by someone with a detected concentration of a body substance to avoid high-risk areas or congested roads; for remote engine disable, for example in extreme cases such as very high concentration of a body substance to allow fleet managers to remotely disable the vehicle to prevent accidents; for person rewards or penalties, for example to implement incentive programs for maintaining clear person records and penalties for violations.

The access control device comprises a communication interface to transmit the access signal to the vehicle, in particular to the access control system of the vehicle. The term "communication interface" may refer to system or protocol that allows devices to exchange data with each other. It may provide a standardized method for transmitting and receiving data, ensuring compatibility and efficient communication between devices. Examples include cable-based communication interfaces like USB or ethernet, or wireless communication interfaces like Wi-Fi, Bluetooth or radio frequency identification (RFID) transmission.

The term "access control system" may refer to a system which can be integrated into a car and serves to exclude unauthorized individuals from accessing and/or using the vehicle. A vehicle may be a car, a motorcycle, a bus, a truck, a tram, a train, a boat, or an airplane. The access control system is suitable for integration into a vehicle. The access control system may be attached to the vehicle, or it may be integrated as component or as part of a component of a vehicle. The access control system may control access to the vehicle or certain functionalities thereof, for example by controlling the doors to open, the vehicle to start, access to the on-board computer, or access to an entertaining system. The access control system comprises a processor. The processor may be dedicated to the access control system, or it may be the on-board computer. The access control system may be communicatively coupled to the on-board computer of the vehicle, for example to use the processor of the on-board computer or to provide access to the on-board computer or functionalities processed by the on-board computer. The access control system comprises a communication interface, for example as defined above, to the portable access control device for receiving an access control signal from the portable access control device.

The access control system generates an unlock signal using the access signal from the access control device. The access signal may either comprise spectroscopic data from which the access control system determines the concentration of a body substance and uses this to generate the unlock signal. The access signal may comprise the concentration of a body substance in case that the access control device has determined it from spectroscopic data. In this case the access control system generates an unlock signal using the concentration of a body substance determined by the access control device. The access control device may only transmit an access signal in case the determination of a body substance has yielded a value which qualifies the person to access the vehicle. In this case, the unlock signal generated by using the concentration of a body substance indirectly, namely by using the access signal which is generated by using the concentration of a body substance.

The term "body substance" may refer to any chemical substance which can be found in a human body, in particular in the skin, blood or interstitial fluid of a human body. The body substance may be indicative of the driver's fitness to drive a vehicle, the body substance may, for example, reduce the concentration of a driver or may be a metabolite of such substance. The body substance may be indicative for a health or fitness condition which compromises the driver's fitness to drive a vehicle, for example a low hydration level or an irregular blood glucose concentration. Body substance may comprise proteins, such as enzymes, antibodies, or hormones; carbohydrates, such as glucose, glycogen, or fructose; lipids, such as triglycerides, cholesterol, and phospholipids; water; nucleic acids, such as DNA or RNA; amino acids, such as alanine, glutamic acid, cysteine; neurotransmitters, such as dopamine, serotonin, and acetylcholine; hormones, such as insulin, estrogen, or testosterone; electrolytes, such as sodium, potassium, or calcium ions; vitamins, such as ascorbic acid, calciferol, cobalamin; metabolites, such as lactate, urea, and creatinine.

Body substance may be an intoxicant or its metabolite including ethanol, opioids, such as heroin, morphine, fentanyl; stimulants, such as amphetamine, methylphenidate, cocaine; benzodiazepines, such as diazepam, or alprazolam; cannabinoids, such as tetrahydrocannabinol (THC); barbiturates, such as phenobarbital; hallucinogens, such as lysergic acid diethylamide (LSD) or psilocybin; antihistamines, such as diphenhydramine; antipsychotics and antidepressants, such as fluoxetine or amitriptyline; muscle relaxants, such as carisoprodol or cyclobenzaprine; pain killers, such as tramadol, codeine, ibuprofen, naproxen, cyclobenzaprine, or methocarbamol.

The concentration of a body substance of the person is determined using the spectroscopic data. The concentration of a body substance of the person may be determined using the spectroscopic data and the person data. The concentration of a body substance of the person may be determined using the spectroscopic data and the environmental data. The concentration of a body substance of the person may be determined using the spectroscopic data, the person data and the environmental data. The concentration may be a numeric value, such as mass ratio or a volume ratio. The ratio may relate to the whole body or parts thereof, for example the skin or the blood. For example, in case of alcohol the blood alcohol concentration may be determined. The concentration may be a categoric value, for example indicating the presence of the body substance or certain value ranges, for example none, low, medium, high.

The term "person data" may refer to data associated with a characteristic of the person such as a physical or chemical characteristic of the person. Person data may refer to any data associated with a characteristic of the person which has been obtained with a method other than spectroscopy. Person data may correlate with the alcohol level of the person. Person data may be personalized data, i.e. specific for a particular person, or it may be data associated with a certain group of people, for example female persons of age 25 to 30. Physical characteristics may comprise thermal characteristics, for example the body temperature, the thermal conductivity or the specific heat capacity of the skin; mechanical characteristics, for example pressure exerted on the spectrometer, compressibility or mechanical elasticity of the skin; optical characteristics, for example the color, refractive index, optical conductivity or absorption coefficients of the skin; electro-magnetic characteristics, for example electrical conductivity, dielectric constant, radio frequency-based permittivity, microwave complex permittivity, millimeter wave complex permittivity, magnetic permittivity or susceptibility of the skin. Chemical characteristics of a person typically refer to the chemical composition of some body tissue like skin, blood or sweat, for example the type and the concentration of certain chemical compounds such as the water content.

The person data may contain or may be a biomarker. The term "biomarker" may refer to a measurable substance, process or characteristic that is indicative of a biological state or condition. A biomarker may refer to a specific molecule, protein, genetic sequence, or other measurable feature that is associated with a particular disease, condition or treatment response. Examples for biomarkers are body dimensions such as size, head circumference, chest girth, abdominal girth, crotch length, arm length; body weight or body mass index; body topology such as face topology, iris structure, finger print, palm topology; muscle measures like muscular strength, muscular endurance, muscular agility and speed, balance, coordination; cardio-vascular measures such as heart rate, heart rate variability, electrocardiogram, blood pressure, blood oxygen; skin measures such as skin conductance, skin impedance, skin moisture level, skin sebum level, skin roughness, skin elasticity, skin pH, skin blood flow, skin sweat rate; blood metabolites such as blood glucose, blood cholesterol, blood triglycerides, blood urea, blood creatinine, blood lactate, blood bilirubin, blood pH; urine metabolites such as urine glucose, urine urea, urine creatinine, urine ketones, urine pH, urine protein content; hormone levels such as thyroid hormone level, insulin level, growth hormone level, cortisol level, estrogen level, progesterone level, testosterone level, prolactin level; drug levels or levels of drug metabolites such as alcohol, amphetamines, opioids, cocaine, marijuana, benzodiazepines, barbiturates.

Person data may be received from sensors other than a spectrometer, for example a thermometer, a scale, a balance, an optical camera, an optical 3D scanner system, conductance or impedance gauge such as a corneometer, a sweat rate monitor or sweat patch, a liquid or gas chromatograph, a mass spectrograph, a nuclear magnetic spectrometer or imager, an electrochemical sensor, an immunoassay, a polymerase chain reaction apparatus. The spectroscopy module may be integrated into a portable device which further comprises sensors from which at least parts of the person data is received.

Person data may be received from a data storage medium. The data storage medium may be part of the access control device or access control system, or it may be a remote storage device, for example a computer system or a cloud system. Person data may be received from a user interface, for example a graphical user interface, to which a user can enter person data, for example from observations. Person data may comprise human characteristics like age, sex, origin, ethnicity; medical history including current and former medications; nutrition such as vegetarian or vegan diet; consumption of stimulants such as caffeine, alcohol, tobacco products, drug; physical activity level such as type of profession, i.e. office job or physically demanding job, kind of sports, average duration of sports, average sleeping hours.

The term "environmental data" may refer to data associated with a characteristic of the surrounding of the person, for example a physical or chemical characteristic of the surrounding of the person. The characteristic of the surrounding of the person may have an influence on the spectroscopic measurement of the person or on the characteristic of the person such as the physical or chemical characteristic of the person. However, environmental data may not comprise an intrinsic characteristic of the person.

Environmental data may comprise sensor data from sensors other than a spectrometer. Environmental data may comprise the location of the person, for example the geolocation such as the GPC coordinates, the height above sea level, distance to a reference point such as the spectrometer, acceleration, orientation with regard to gravity; weather conditions such air temperature, air pressure, air humidity, wind speed, wind direction, ambient light intensity; time or date; air pollutant levels like CO₂ concentration, CO concentration, ozone concentration, nitrogen oxide concentration, sulfur dioxide concentration, fine dust concentration, volatile organic compounds level.

Sensor data may have been recorded by a sensor capable of determining the sensor data. The sensor may be integrated into the spectrometer. The spectroscopy module may be integrated into a portable device which further comprises sensors from which at least parts of the environmental data is received. The sensor may be communicatively coupled to the spectrometer, for example via a wireless communication or via internet. Examples for sensors may be a GPC receiver, an accelerometer, a gyroscope, an altimeter, a goniometer, a distance sensor like a time-of-flight sensor, a radar or a LiDaR, a pressure sensor such as a MEMS sensor, a piezo sensor or a capacitive sensor, a magnetometer, a barometer, a light sensor, a thermometer, a gas sensor.

Environmental data may comprise data associated with the spectrometer module, for example a spectrometer module ID, a version number of the spectrometer module, the spectrometer module settings, the temperature of the spectrometer module, the age of the spectrometer module, time since the last calibration was performed, age of the illumination source, number of measurements the spectrometer module has already performed in its lifetime or within a certain time such as the last week or the last month. Environmental data may further comprise data associated with the spectroscopic measurement of the person, for example the sampling time, the illumination strength with which the spectrometer illuminates the person, or the distance of the person to the spectrometer.

Environmental data may be received from a data storage medium. The data storage medium may be part of the access control device or access control system, or it may be a remote storage device, for example a computer system or a cloud system. Environmental data may be received from a database, for example from a database on a remote storage system, in response to a request containing time and/or geographic location. A remote storage system may refer to a system which is far from the person of the measurement, for example a cloud server or a database server. For example, a request containing the GPS coordinates of the person and the time of the spectroscopic measurement may be sent to a cloud server having a weather database. The cloud server may in response to the request send weather data corresponding to the time and location of the request.

Determining the concentration of a body substance may involve a reference spectrum. The reference spectrum may be a spectrum measured when the concentration of the body substance is known. The reference spectrum may be a spectrum of a reference person, or an average over spectra from different persons or it may be a personalized spectrum of person seeking access to the vehicle. The reference spectrum may hence be a personalized spectrum, i.e. a spectrum particular for the person seeking access to the vehicle. The personalized spectrum may be retrieved using an identifier for the person The identifier indicating the identify of the person seeking access to the vehicle may be determined using an identifier of the access control device, in particular if the access control device is exclusively used by a single person. The identity of the person seeking access to the vehicle may be determined by a user input, for example a personalized password or a biometric recognition, for example a fingerprint or a face recognition. The reference spectrum may be measured with the spectroscopic module. The spectroscopic module may be triggered to measure a reference spectrum if a reference condition is fulfilled. The term "reference condition" may refer to a condition or a set of conditions at which a spectroscopic measurement shall be triggered in order to obtain a reference spectrum.

Alternatively, the reference spectrum may be obtained from a database. The reference spectrum may have been recorded under controlled conditions, for example in the course of enrollment, and stored to the database. The database may be stored in a memory comprised in the access control device or access control system or it may be stored in a cloud service. The combined spectrum may be compared to the reference spectrum. For example, a difference spectrum may be determined by subtracting the reference spectrum from the combined spectrum. Alternatively, the spectra are subject to principle component analysis and the principle components are used for comparison and/or combination. In this way, the influence of background can be reduced. Also, outliers can be identified.

The concentration of a body substance of the person may be determined by employing a chemometric model. The term "chemometric model" may refer to a model which is parameterized to receive spectroscopic data as input and output the concentration of a body substance. The chemometric model may be parameterized to receive spectroscopic data and person data as input and output the concentration of a body substance. The chemometric model may be parameterized to receive spectroscopic data and environmental data as input and output the concentration of a body substance. The chemometric model may be parameterized to receive spectroscopic data, person data and environmental data as input and output the concentration of a body substance. The chemometric model may be parameterized to receive spectroscopic data as input and output an intermediate concentration of a body substance. The intermediate concentration of a body substance may be adjusted or corrected using the person data and/or the environmental data, for example by employing a refining model. The refining model may be a data-driven model which may be trained with historic data for adjusting or correcting the intermediate concentration of a body substance. A refining model may be a multivariate linear or polynomial regression model, or it may be an artificial neural network.

A chemometric model may comprise a pre-processing method and a machine learning model to obtain the concentration of a body substance. A chemometric model may comprise a pre-processing method, a feature selection filter and a machine learning model. If the chemometric model comprises two or more partial chemometric models, each partial chemometric model may comprise a separate pre-processing method, a feature selection filter and a machine learning model. Alternatively, the partial models may use the same pre-processing method or feature selection filter.

The term "pre-processing" may refer to a method to reduce or eliminate interferences from a spectrum such as stray light, noise or baseline drift to enhance the subsequent machine learning. Hence, the pre-processing method may be applied before the machine learning method. Pre-processing may include one or more of baseline correction, scatter correction, smoothing, scaling, aggregation.

The term "machine learning method" may refer to a model which translates spectra into corresponding person data. The machine learning method hence may use a spectrum as input and derive person data therefrom. The machine learning method may be considered as an integral part of the chemometric model. Machine learning methods may be supervised, semi-supervised or unsupervised. Machine learning methods may include multivariate calibration, classification, pattern recognition, clustering, ensemble methods, neural nets and deep learning, or multivariate curve resolution.

The term "feature selection filter" may refer to a method to select those parts of the spectrum with a correlation to the person data. A feature selection filter may facilitate the machine learning method of the chemometric model and thus avoid overfitting and reduce the number of required training datasets. A feature selection filter may use a spectrum as input, remove all unselected parts and output a spectrum with only the selected parts left. Hence, the output of the feature selection filter may be a spectrum in form of a vector of lower dimensionality than the input vector. The output of the feature selection filter can be used as input for the machine learning method. Hence, the feature selection filter may be applied before the machine learning method. The input of the feature selection filter may be the received spectrum or it may be the pre-processed spectrum, preferably the pre-processed spectrum. Hence, the feature selection filter may be applied after the pre-processing method.

A chemometric model may be or may contain a data-driven model. The chemometric model may be a trained data-driven model. Training may comprise adjusting parameters of the chemometric model such that the output of the chemometric model most closely fits to the provided training data. Often, training comprises minimizing a loss or cost function, for example a least mean square value of chemometric model output to provided training data. The complete set of training data may be used for training or parts thereof. Parts of the received training data may be used for training and the remainder may be used for determining the prediction accuracy of the trained chemometric model. Alternatively, cross-validation can be applied, for example K-fold cross-validation, leave-one-out cross-validation, stratified cross-validation.

The access control system comprises a communication interface, for example as defined above, to for transferring the unlock signal to the locking mechanism. The term "locking mechanism" may refer to a mechanism which can physically exclude access. The locking mechanism may be hardware, for example a mechanic lock, or a software-based lock, for example encryption or a mechanism allowing the execution of an app only to authorized persons. Hence, the access control system may output the unlock signal through the communication interface.

The access control device and the access control system comprise a processor. The term "processor" may refer to a logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math co-processor or a numeric co-processor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a micro-processor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) and/or one or more tensor processing unit (TPU) and/or one or more chip, such as a dedicated machine learning optimized chip, or the like. The processor specifically may be configured, such as by software programming, for performing one or more evaluation operations. At least one or any component of a computer program configured for performing the authentication process may be executed by the processing device. Alternatively or in addition, the processor may be or may comprise a connection interface. The connection interface may be configured to transfer data from the device to a remote device; or vice versa. At least one or any component of a computer program configured for performing the authentication process may be executed by the remote device.

The present invention further relates to a non-transient computer-readable medium including instructions that, when executed by one or more processors, cause the one or more processors to perform the method according to the present invention. The term "computer-readable data medium" may refer to any suitable data storage device or computer readable memory on which is stored one or more sets of instructions (for example software) embodying any one or more of the methodologies or functions described herein. The instructions may also reside, completely or at least partially, within the main memory and/or within the processor during execution thereof by the computer, main memory, and processing device, which may constitute computer-readable storage media. The instructions may further be transmitted or received over a network via a network interface device. Computer-readable data medium include hard drives, for example on a server, USB storage device, CD, DVD or Blue-ray discs. The computer program may contain all functionalities and data required for execution of the method according to the present invention or it may provide interfaces to have parts of the method processed on remote systems, for example on a cloud system.

### Brief Description of the Figures

- Figure 1: illustrates an example of the access control system for a vehicle.
- Figure 2: illustrates an example for a spectroscopy module.
- Figure 3: illustrates two examples for the method of the present invention.

Figure 4 illustrates two examples of how the concentration of a body substance can be determined from spectroscopic data, person data and/or environmental data.

### Description of Embodiments

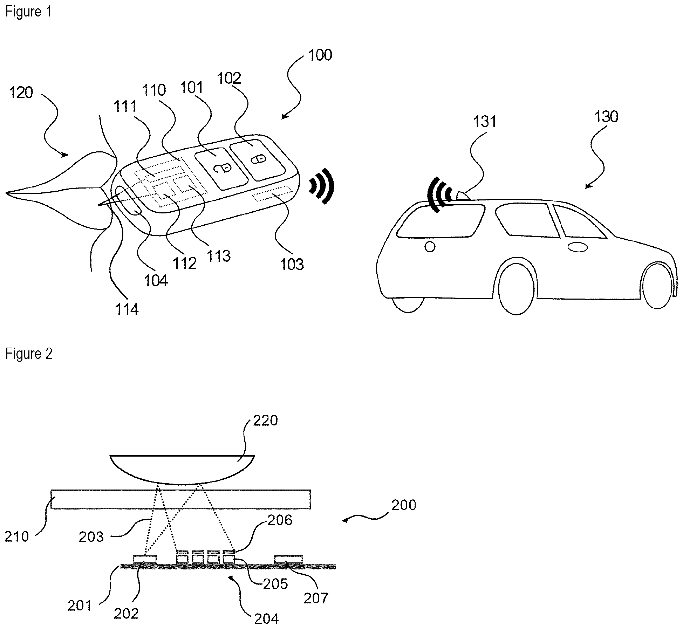

Figure 1 illustrates an example of the access control system for a vehicle. A portable access control device 100 may be a key, for example a wireless car key. The key may comprise an open button 101 and a close button 102. When pressing the open button 101 a person may indicate its desire to seek access to a vehicle 130, for example a car. The key 100 may comprise a spectroscopy module 110. In response to pressing the open button 101 the spectroscopy module 110 may be triggered to execute a measurement. The spectroscopy module 110 may comprise a light emitting element 111, for example an LED. The light emitting element 111 may emit a light beam 114, for example infrared light in the range of 750 nm to 2.5 µm. The light beam 114 may exit the key via a transparent window 104 and hit a body part of the person, for example his lips 120. The lips 120 may reflect the light beam 114 through the transparent window 104. The light beam 114 may impinge on a photosensor 112 of the spectroscopy module 110. The photosensor 112 may generate electrical signals which are processes by a processor 113, for example an ASIC or a microcontroller, into spectroscopic data. An access signal may be generated comprising the spectroscopic data. The access signal may be transmitted by transmitter 103, for example by a radio frequency signal in the range of 300 to 400 MHz.

The access signal may be received by vehicle 130 via a receiver 131. The receiver 131 may send the access signal to a processor, for example a board computer of the vehicle 130. The processor may determine a blood alcohol concentration of the person seeking access to the vehicle by inputting the spectroscopic data to a chemometric model which is parametrized to receive spectroscopic data as input and output the blood alcohol concentration. The processor may identify the person seeking access to the vehicle, for example by using the identifier of the key 100 contained in the access signal. Personal data, for example a personal reference spectrum, may be used for determining the blood alcohol concentration, wherein the personal data may be stored in a memory in the on-board computer system and may be accessed using the person identification, for example the identifier of the key 100. The determined blood alcohol concentration may be used to determine the fitness to drive of the person seeking access to the vehicle. For example, the fitness to drive may be determined by comparing the blood alcohol concentration with a predefined threshold, for example 0.05 %. If the blood alcohol concentration is blow the threshold, the processor may generate an unlock signal which may be transferred to a locking mechanism of the vehicle. The locking mechanism of the vehicle may in response to receiving the unlock signal unlock the vehicle, for example by opening the door locks. Otherwise, access to the vehicle may be denied.

Figure 2 illustrates an example for a spectroscopy module. The spectroscopy module may be suitable to acquire spectroscopic data of the driver, for example from his lips 220. The spectroscopy module may comprise a spectroscopic module 200. The spectroscopic module 200 may comprise a substrate 201, for example a printed circuit board (PCB). The spectroscopic module 200 may comprise a light emitting element 202, for example an LED. The LED may emit light of a desired wavelength, for example infrared light in the range of 750 nm to 2.5 µm. The light emitting element 202 may emit a light ray 203 directed towards the finger 220 of the driver. Between the spectroscopic module 200 and the finger 220, there may be a cover 210 which is at least partially transparent to the light emitted by the light emitting element 202. The cover 210 may be a sheet of glass or a polymer like polycarbonate or polymethyl methacrylate (PMMA). The cover 210 may also be a transparent display, for example the display of a control panel or a multimedia system.

The spectroscopic module 200 may comprise a set of photosensors 204 which may be mounted on the substrate 201. The set of photosensors 204 may comprise an array of photosensors, for example a 3 times 3 array. Each photosensor 205 may be sensitive to light at the wavelength range emitted by the light emitting element 202. The light ray 203 may impinge on the set of photosensors 204 after having penetrated into the finger 220. Each photosensor 205 may be covered with an optical filter 206. The optical filters 206 may be chosen to let pass light at different wavelengths, so each photosensor 205 receives a different wavelength range of light. The photosensor 205 may comprise a photosensitive material, for example a photoconductor like lead sulfide (PbS). The photosensor may generate an electric signal depending on the light intensity of the light impinging on the photosensor 205.

The spectroscopic module 200 may comprise a processor 207 which may be mounted on the substrate 201. The processor 207 may be operatively coupled to the light source 202 and the photodetectors 205, for example via electric conductors on the PCB. The processor 207 may be a microcontroller configured to control the light emitting element 202, for example to switch it on during the measurement and switch it off afterwards. The processor 207 may be a microcontroller configured to receive the electric signal from the photosensors 205 and convert them into digital signals by analog-to-digital conversion. The processor may thus generate spectroscopic data which may either be further processed to determine the concentration of a body substance of the driver or it may forward the spectroscopic data to an electronic control unit (ECU) of the vehicle or the on-board computer of the vehicle for determining the concentration of a body substance of the driver.

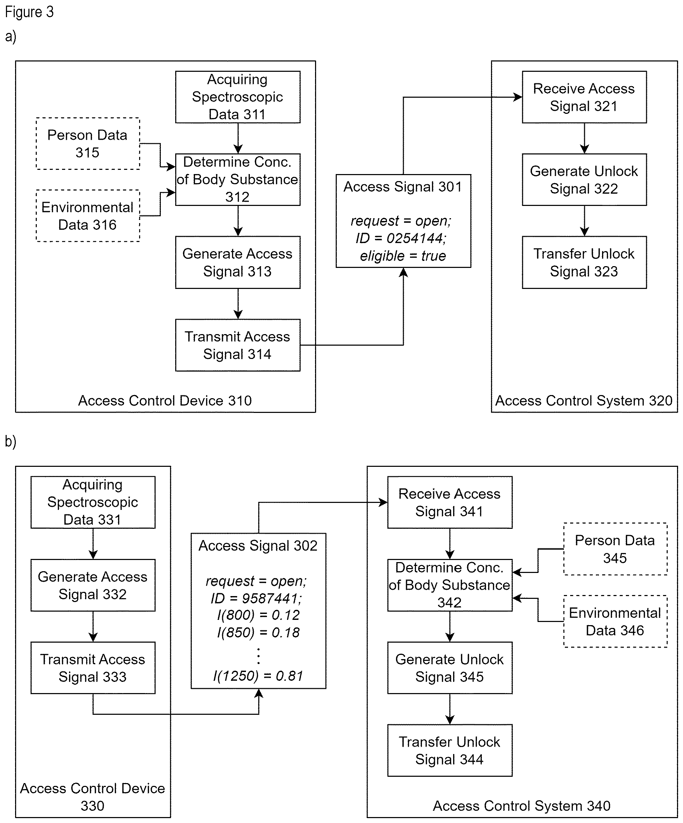

Figure 3 illustrates two examples for the method of the present invention. In figure 3a the access control device 310 determines the concentration of the body substance and transmits an access signal 301 to the access control system 320, while in figure 3b the access control device 330 transmits the spectroscopic data as part of the access signal 302 to the access control system 340 which determines the concentration of the body substance.

Figure 3a illustrates an example in which the access control device 310 may be equipped with reasonably high computation power, for example, the access control device 310 may be a smartphone. The access control device 310 may receive an access request, for example a person presses a respective button of the access control device 310 or enters a corresponding command. In response to the access request spectroscopic data 311 may be acquired. This may be achieved by triggering a spectroscopic module integrated into the access control device 310. The spectroscopic module may record a spectrum of the person having submitted the access request. The access control device 310 may determine the concentration of a body substance 312, for example alcohol or THC. Such determination may involve person data 315, for example age or sex of the person, and/or environmental data 316, for example the air temperature around the person. The access control device 310 may generate 313 an access signal 301. The access signal 301 may comprise a request flag indicating that access is requested, for example by setting it to "open". The access signal 301 may further comprise an identifier. The identifier may identify the access control device 310 and/or the person having initiated the access request. The access signal 301 may comprise an eligibility flag indicating that the person is eligible to access the vehicle. The eligibility flag may be set as a result of comparing the determined concentration of the body substance to a threshold. For example, the hydration level may be determined and compared to a threshold. If the hydration level is above the threshold, the eligibility flag may be set to "true", otherwise to "false". It is also possible that the access signal 301 is only generated if the person is eligible, otherwise no access signal 301 may be generated. The access signal 301 may be encrypted to avoid information loss to unauthorized listeners or by electronically signed to ensure that the access signal 301 really originates from access control device 310. The access control device 310 may transmit the access signal 301 to the access control system in the vehicle 320, for example by Wi-Fi.

The access control system 320 may receive 321 the access signal 301. The access control system 320 may verify the authenticity of the access signal, for example by checking the identifier, the electronic signature, or by decrypting the access signal 301, depending on the case. The access control system 320 may generate an unlock signal 322 using the access signal 301. The unlock signal 322 may only be generated if the access signal 301 is found to be authentic and contains the proper access request. The unlock signal may be transferred to 323 to the locking mechanism, for example causing a mechanic or electronic lock to open.

Figure 3b illustrates an example in which the access control device 330 may be equipped with little computation power, for example, the access control device 330 may be a key fob. The access control device 330 may receive an access request, for example a person presses a respective button of the access control device 330. In response to the access request spectroscopic data 331 may be acquired. This may be achieved by triggering a spectroscopic module integrated into the access control device 330. The spectroscopic module may record a spectrum of the person having submitted the access request. The access control device 330 may generate 332 an access signal 302. The access signal 302 may comprise a request flag indicating that access is requested, for example by setting it to "open". The access signal 302 may further comprise an identifier. The identifier may identify the access control device 330 and/or the person having initiated the access request. The access signal 302 may comprise spectroscopic data, for example a matrix of values indicating a wavelength and an absorption or transmission value. The access control device 330 may transmit 333 the access signal 302 to an access control system 340 in a vehicle, for example by RFID.

The access control system 340 may receive 341 the access signal 302. The access control system 340 may use the access signal 302 to determine the concentration of a body substance 342, for example blood alcohol. This may be achieved by extracting spectroscopic data from the access signal 302 and input it into a chemometric model. The chemometric model may output the concentration of the body substance. The access control system 340 may generate an unlock signal, for example as described above. The unlock signal may be transferred 344 to a locking mechanism, for example to open the doors of the vehicle.

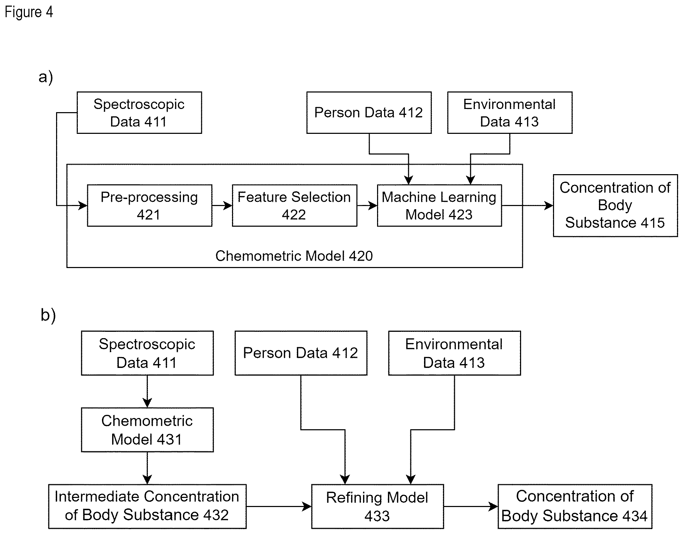

Figure 4 illustrates two examples of how the concentration of a body substance can be determined from spectroscopic data, person data and/or environmental data. In Figure 2a spectroscopic data 411 may be input to a chemometric model comprising pre-processing 421, feature selection 422 and a machine learning model 423. Spectroscopic data 411 may comprise a spectrum, for example a near infrared spectrum, obtained from a measurement with a spectrometer. Pre-processing 421 may comprise baseline correction, for example first-order derivation, scatter correction, for example standard normal variate, smoothing, for example moving average filtering, scaling, for example Pareto scaling, aggregation, for example spatial median. The pre-processed spectroscopic data may subsequently undergo feature selection 422. Feature selection 422 may reduce the dimensionality of the spectroscopic data 411, so training the machine learning model 423 requires less training data. Feature selection 422 may for example involve principle component regression (PCR). The thus pre-processed and feature-selected spectroscopic data may be passed as input to a machine leaning model 423, for example an artificial neural network. The machine leaning model 423 may be parametrized to further receive the person data 412 and the environmental data 413 as further input. The person data 412 and the environmental data 413 may be pre-processed before inputting into the machine learning model 413, for example to adjust the format and the units of the data. The machine leaning model 423 may be trained with historic data comprising spectroscopic data, person data and environmental data. The machine leaning model 423 may output the concentration of a body substance 415, for example its quantity or concentration. Using a chemometric model which uses spectroscopic data, person data and environmental data as input has the advantage that complex interplays between spectroscopic data, person data and environmental data can be taken into account.

Figure 4b shows an alternative example for determination of a concentration of a body substance from spectroscopic data, person data and/or environmental data. Spectroscopic data 411 may be input to a chemometric model 431 which outputs an intermediate concentration of a body substance 432. The chemometric model 431 may not be parametrized to take person data 412 and/or environmental data 413 into account. Hence, the intermediate concentration of a body substance 432 only depends on the spectroscopic data 432. In order to obtain the desired concentration of a body substance 434, a refining model 433 may be employed. The refining model 433 may be parametrized to receive the intermediate concentration of a body substance 432, the person data 412 and the environmental data 413 and to output the concentration of a body substance 434. The refining model 433 may comprise to sub-models, one which processes the person data 412 and one which processes the environmental data 413. For example, the first sub-model may receive the intermediate concentration of a body substance 432 and the person data 412 as input and output a refined concentration of a body substance. A second sub-model may use the refined concentration of a body substance and the environmental data 413 as input and output the concentration of a body substance 434. The refining model 433 may be a multivariate polynomial regression model which adjusts the intermediate concentration of a body substance 432 according to the person data 412 and the environmental data 413 to arrive at the concentration of a body substance 434. A refining model 433 has the advantage that the chemometric model 431 does not need a retraining for new person data types or environmental data types.

The present disclosure has been described in conjunction with preferred embodiments and examples as well. However, other variations can be understood and effected by those persons skilled in the art and practicing the claimed invention, from the studies of the drawings, this disclosure and the claims.

Any steps presented herein can be performed in any order. The methods disclosed herein are not limited to a specific order of these steps. It is also not required that the different steps are per-formed at a certain place or in a certain computing node of a distributed system, i.e. each of the steps may be performed at different computing nodes using different equipment/data processing.

As used herein "determining" also includes "initiating or causing to determine", "generating" also includes "initiating and/or causing to generate" and "providing" also includes "initiating or causing to determine, generate, select, send and/or receive". "initiating or causing to perform an action" includes any processing signal that triggers a computing node or device to perform the respective action.

In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation. In the claims as well as in the description the word "comprising" or "including" or similar wording does not exclude other elements or steps and shall not be construed limiting to the elements or steps lined out. The indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation or further elements may be included.

Providing in the scope of this disclosure may include any interface configured to provide data. This may include an application programming interface, a human-machine interface such as a display and/or a software module interface. Providing may include communication of data or sub-mission of data to the interface, in particular display to a user or use of the data by the receiving node, entity or interface.

Various units, circuits, entities, nodes or other computing components may be described as "configured to" perform a task or tasks. Configured to shall recite structure meaning "having circuitry that" performs the task or tasks on operation. The units, circuits, entities, nodes or other computing components can be configured to perform the task even when the unit/circuit/component is not operating. The units, circuits, entities, nodes or other computing components that form the structure corresponding to "configured to" may include hardware circuits and/or memory storing program instructions executable to implement the operation. The units, circuits, entities, nodes or other computing components may be described as performing a task or tasks, for convenience in the description. Such descriptions shall be interpreted as including the phrase "configured to." Any recitation of "configured to" is expressly intended not to invoke 35 U.S.C. § 112(f) interpretation.

In general, the methods, apparatuses, systems, computer elements, nodes or other computing components described herein may include memory, software components and hardware components. The memory can include volatile memory such as static or dynamic random-access memory and/or nonvolatile memory such as optical or magnetic disk storage, flash memory, programmable read-only memories, etc. The hardware components may include any combination of combinatorial logic circuitry, clocked storage devices such as flops, registers, latches, etc., finite state machines, memory such as static random-access memory or embedded dynamic random-access memory, custom designed circuitry, programmable logic arrays, etc.

Any disclosure and embodiments described herein relate to the methods, the systems, apparatuses, devices, chemicals, materials, computer program elements lined out above and vice versa. Advantageously, the benefits provided by any of the embodiments and examples equally apply to all other embodiments and examples and vice versa. All terms and definitions used herein are understood broadly and have their general meaning.

## Claims

1. A portable access control device for a vehicle comprising:
a) a spectroscopy module for acquiring spectroscopic data of a person,
b) a processor configured to generate an access signal using the spectroscopic data, and
c) a communication interface to transmit the access signal to the vehicle.

2. The portable access control device according to claim 1, wherein the processor is configured to determine a concentration of a body substance from the spectroscopic data and to generate an access signal using the concentration of the body substance.

3. The portable access control device according to claim 2, wherein the body substance is an intoxicant like blood alcohol.

4. The portable access control device according to any of the claims 1 to 3, wherein portable access control device is a key fob or a smartphone.

5. The portable access control device according to any of the claims 1 to 4, wherein the communication interface is a wireless communication interface.

6. The portable access control device according to any of the claims 1 to 5, wherein the spectroscopy module is positioned such that the lips of the person can be measured.

7. The portable access control device according to any of the claims 1 to 6, wherein the spectroscopy module comprises a plurality of photosensors, wherein each photosensor is positioned behind an optical filter with regard to the beam path.

8. An access control system for integration into a vehicle comprising:
a) a communication interface to receive an access signal from a portable access control device, wherein the access signal comprises data derived from a spectroscopic measurement,
b) a processor configured to generate an unlock signal using the access signal, wherein the unlock signal generation involves a determination of the eligibility to drive of the person seeking access to the vehicle from the access signal, and
c) a communication interface for transferring the unlock signal to a locking mechanism of the vehicle.

9. The access control system according to claim 8, wherein the processor is configured to determine a concentration of a body substance from spectroscopic data comprised in the access signal and to generate an unlock signal using the concentration of the body substance.

10. The access control system according to claim 8 or 9, wherein the access control system is communicatively coupled to the on-board computer of the vehicle.

11. A method for controlling access to a vehicle comprising:
a) receiving spectroscopic data of a person seeking access to the vehicle from a spectroscopy module integrated into a portable access control device,
b) determining a concentration of a body substance from the spectroscopic data,
c) generating an unlock signal using the concentration of the body substance, and
d) transferring the unlock signal to a locking mechanism of the vehicle.

12. The method according to claim 11, wherein person data is additionally used to determine the concentration of the body substance, wherein person data refers to data associated with a characteristic of the person.

13. The method according to claim 11 or 12, wherein environmental data is additionally used to determine the concentration of the body substance, wherein environmental data refers to data associated with a characteristic of surrounding of the person.

14. The method according to any of the claims 11 to 13, wherein the identity of the person is determined and wherein a personalized reference spectrum of the identified person is used to determine the concentration of the body substance.

15. A non-transient computer-readable medium including instructions that, when executed by one or more processors, cause the one or more processors to perform a method comprising:
a) receiving spectroscopic data of a person seeking access to the vehicle from a spectroscopy module integrated into a portable access control device,
b) determining a concentration of a body substance from the spectroscopic data,
c) generating an unlock signal using the concentration of the body substance, and
d) transferring the unlock signal to a locking mechanism of the vehicle.
